# EUROPEAN PATENT SPECIFICATION

(11) **EP 2 732 765 B1**
(45) Date of publication and mention of the grant of the patent: **20.04.2016**
(21) Application number: 13193337.6
(22) Date of filing: 18.11.2013
(51) Int. Cl.: A61B 6/12, A61B 5/06, A61B 6/00, G06T 7/20, A61B 90/00

(54) **Patient movement compensation in intra-body probe tracking systems**
Patientenbewegungskompensation in intrakorporalen Sondenverfolgungssystemen
Compensation de mouvement de patient dans des systèmes de suivi de sonde intracorporelle

(30) Priority: 19.11.2012 US 201213680551
(43) Date of publication of application: 21.05.2014
(73) Proprietor: Biosense Webster (Israel), Ltd., Yokneam 20692 (IL)
(72) Inventor: Shalgi, Avi, Irvine, CA California 92604 (US); Amit, Matityahu, 44862 Zur-Yigal (IL); Bar-Tal, Meir, 32240 Haifa (IL); Hayam, Gal, 36501 Tivon (IL)
(74) Representative: Carpmaels & Ransford LLP

(56) References cited:
- US-A1- 2011 160 569
- AZIZIAN MAHDI ET AL: "Data fusion for catheter tracking using Kalman filtering: applications in robot-assisted catheter insertion", MEDICAL IMAGING 2011: VISUALIZATION, IMAGE-GUIDED PROCEDURES, AND MODELING, SPIE, 1000 20TH ST. BELLINGHAM WA 98225-6705 USA, vol. 7964, no. 1, 3 March 2011 (2011-03-03), pages 1-11, XP060008092, DOI: 10.1117/12.878148 [retrieved on 2011-03-01]
- R. MANZKE ET AL: "<title>Integration of real-time x-ray fluoroscopy, rotational x-ray imaging, and real-time catheter tracking for improved navigation in interventional cardiac electrophysiology procedures</title>", PROCEEDINGS OF SPIE, vol. 6141, 2 March 2006 (2006-03-02), pages 614112-614112-9, XP055098982, ISSN: 0277-786X, DOI: 10.1117/12.651715

## Description

### FIELD OF THE INVENTION

The present invention relates generally to intra-body probe tracking, and particularly to methods for compensating patient movements during intra-body probe tracking.

### BACKGROUND OF THE INVENTION

Methods for locating the position of medical devices in the human body by magnetic and imaging procedures are known in the prior art. For example, U.S. Patent Application Publication 2011/0160569 describes a method and a system for mapping a volume of an anatomical structure. The system includes a processor for computing a contour of a medical device as a function of positional and/or shape constraints, and to translate the contour into known and virtual 3D positions.

U.S. Patent Application Publication 2011/0054308 describes a system for superimposing virtual anatomical landmarks on an image. The system includes a medical positioning system (MPS) for producing location readings with respect to points within a region of interest in accordance with an output of a location sensor disposed in a medical device.

U.S. Patent 7,706,860 describes methods and systems for navigating medical devices. A patient is imaged while a medical device is moved within the patient. A position (e.g., a location and/or orientation) of the moving medical device is detected within a coordinate system (e.g., a three-dimensional coordinate system), a position of the imaging field of view relative to the patient is adjusted based on the detected medical device position, such that the relevant tissue region of the patient is within the field of view.

U.S. Patent 4,662,379 describes a method of imaging a blood vessel such as a coronary artery which includes the steps of a dual energy providing radiation source and a radiation detector on opposing sides of a target area and at a plurality of angular positions through the target area.

U.S. Patent Application Publication 2010/0145197 describes a device and method for generating a motion-corrected 3D image of a cyclically moving object by means of an ultrasound probe, comprising the steps of providing at least one 3D reference image of the object, the 3D reference image showing the object substantially at one particular phase in its cyclic movement; acquiring a set of sub-images of the object by sweeping the ultrasound probe over the moving object; registering at least two, preferably all, of the sub-images with the 3D reference image, thereby generating at least two motion-corrected sub-images; and reconstructing a motion-corrected 3D image from the motion-corrected sub-images. The invention is also directed to a corresponding device, computer program, and digital storage medium.

'Data Fusion for Catheter Tracking using Kalman Filtering: Applications in Robot-Assisted Catheter Insertion', Mahdi Azizian et al., Proceedings of SPIE vol. 7964 (DOI: 10.1117/12.878148) describes a method and device for combining fluoroscopy and magnetic tracking to compensate for deficiencies in each technique.

'Integration of real-time X-ray fluoroscopy, rotational X-ray imaging and real-time catheter tracking for improved navigation in interventional cardiac electrophysiology procedures', R. Manzke et al., Proceedings of SPIE vol. 6141 (DOI: 10.1117/12.651715) describes a method of combining rotational and real time X-ray imaging with real-time catheter tracking for improved catheter guidance.

### SUMMARY OF THE INVENTION

An embodiment of the present invention that is described herein provides a method including receiving a position of an intra-body probe inserted into an organ of a living body in a first coordinate system. Fluoroscopic images of the body are received. A movement of the body in the fluoroscopic images is measured in a second coordinate system. The received position of the intra-body probe in the first coordinate system is corrected using the movement identified in the second coordinate system.

In some embodiments, measuring the movement includes identifying a hard tissue of the body in the fluoroscopic images, and assessing the movement of the hard tissue between a first fluoroscopic image and a subsequent fluoroscopic image. In other embodiments, assessing the movement includes defining multiple anchor points on the hard tissue, and assessing the movement of the anchor points between the first fluoroscopic image and the subsequent fluoroscopic image. In yet other embodiments, correcting the received position of the intra-body probe includes applying the movement of the anchor points, which was assessed in the second coordinate system, to the received position of the intra-body probe in the first coordinate system.

In some embodiments, receiving the position includes defining the first coordinate system relative to one or more body patch sensors using a magnetic tracking system, and wherein receiving the fluoroscopic images includes identifying the body patch sensors in the fluoroscopic images and defining the second coordinate system relative to the identified body patch sensors. In other embodiments, correcting the received position using the measured movement of the body is performed upon determining that the movement of one or more body patch sensors, which are disposed on the body and detected by a magnetic tracking system, exceeds a predefined threshold.

In some embodiments, receiving the fluoroscopic images includes, for a given fluoroscopic image, receiving two or more fluoroscopic sub-images acquired at different angles in the second coordinate system. In other embodiments, measuring the movement of the body includes constructing first and second three-dimensional models of hard tissue of the body from respective first and second fluoroscopic images, and measuring the movement of the hard tissue between the first and second three-dimensional models of the hard tissue. In yet other embodiments, the method also includes tracking a movement of heart anchor points, so as to improve an accuracy in correcting the received position of the intra-body probe.

There is additionally provided, in accordance with an embodiment of the invention, an apparatus including an interface and a processor. The interface is configured to receive a position of an intra-body probe inserted into an organ of a living body in a first coordinate system, and to receive fluoroscopic images of the living body. The processor is configured to measure a movement of the body in the fluoroscopic images in a second coordinate system, and to correct the received position of the intra-body probe in the first coordinate system using the movement identified in the second coordinate system.

There is also provided, in accordance with an embodiment of the invention, an apparatus including a magnetic intra-body probe tracking system, a fluoroscopic imaging system, and a patient movement compensation system. The patient movement compensation system is configured to receive from the magnetic intra-body probe tracking system a position of the intra-body probe inserted into an organ of a living body in a first coordinate system, to receive from the fluoroscopic imaging system fluoroscopic images of the living body in a second coordinate system, to measure a movement of the body in the fluoroscopic images in the second coordinate system, and to correct the received position of the intra-body probe in the first coordinate system using the identified movement.

The present invention will be more fully understood from the following detailed description of the embodiments thereof, taken together with the drawings in which:

### BRIEF DESCRIPTION OF THE DRAWINGS

Fig. 1 is a schematic, pictorial illustration of an intra-body probe tracking system, in accordance with an embodiment of the present invention;
Fig. 2 is a block diagram that schematically illustrates an intra-body probe tracking system, in accordance with an embodiment of the present invention;
Figs. 3A and 3B are diagrams illustrating a translation and a rotation of a hard tissue utilized in an intra-body probe tracking system, in accordance with an embodiment of the present invention; and
Fig. 4 is a flow chart that schematically illustrates a method for compensating for patient movement in an intra-body probe tracking system, in accordance with an embodiment of the present invention.

### DETAILED DESCRIPTION OF EMBODIMENTS

### OVERVIEW

Embodiments of the present invention that are described herein provide improved methods and systems for compensating for patient movements during the tracking of an intra-body probe, such as a catheter, within an organ or cavity of a living body.

In an example embodiment, a magnetic tracking system (MTS) is used to track the position of the distal tip of the catheter during navigation in the body, in combination with body patch probes that are placed on surface of the patient whose positions are also tracked by the MTS. The MTS measures the positions of the body patch probes, which are then used as a frame of reference for the position measurements of the catheter distal tip.

If the patient moves during the procedure, the MTS frame of reference defined by the body patch probes will be shifted, and the measurement of the location of the catheter distal tip will become inaccurate. The MTS frame of reference may be corrected by tracking the movement of the body probes. However, if the patient movement is too excessive, the MTS may not be able to compensate for it. It is possible in principle to recalibrate the reference frame of the MTS in order to compensate for the movement. The recalibration cycle, however, is time consuming and disruptive to the procedure.

The methods and systems described herein compensate for patient movements by processing fluoroscopic images acquired by a fluoroscope imaging system (FIS). In an example embodiment, if the patient movements exceed the MTS movement threshold, a patient movement compensation system (PMCS) uses successive fluoroscopic images to measure the patient movement by detecting the movement of hard tissue, such as the bones in the rib cage.

The PMCS typically receives both the position of the distal tip of the catheter and the body patches from the MTS, as well as the fluoroscopic images of the patient from the FIS. A processor of the PMCS is configured to assign anchor points on landmarks on the hard tissue of a first fluoroscopic image and to measure the position of the anchor points on one or more successive fluoroscopic images. The patient movement is typically measured by computing a translation vector and rotation of the movement of the assigned anchor points. The translation and rotation are then applied to resynchronize the frame of reference of the MTS restoring the accuracy of the catheter tracking without the need for an MTS recalibration cycle.

### SYSTEM DESCRIPTION

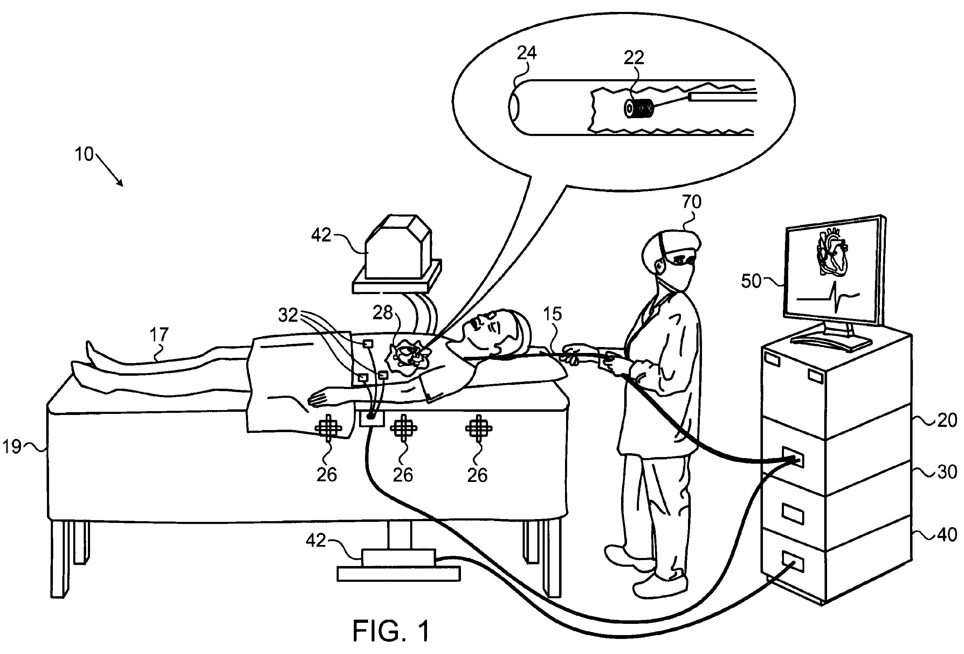

Fig. 1 is a schematic, pictorial illustration of an intra-body probe tracking system 10, in accordance with an embodiment of the present invention. A catheter 15 is connected to a catheter magnetic tracking system (MTS) 20 and percutaneously inserted into a living body 17 of a patient laying on a gurney 19. Catheter 15 comprises a magnetic position sensor 22 at a distal tip 24, which is navigated into an organ, such as a human heart 28.

One or more magnetic skin patch probe sensors 32 are attached to the surface of patient body 17 and connected to MTS 20. One or more magnetic field generators 26 create a magnetic field through the body of the patient, which induce signals in catheter sensor 22 and body patch sensors 32, which are typically placed on the patient's back. The induced signals are used by MTS 20 to track and locate the position of sensor 22 in the catheter distal tip. The tracked position of the catheter distal tip is typically displayed to an operator 70 on an output display monitor 50.

Catheter position sensor 22 typically comprises one or more miniaturized coil sensors. In a tri-axial sensor (TAS) used in magnetic tracking systems, such as the CARTO system (Biosense Webster, Diamond Bar, CA), three coils are orthogonally configured at the distal tip of the catheter to create a received signal in response to the magnetic field in order to measure the local vector magnetic field at the distal tip by using the induced electrical signals in the sensor coils. In other systems, sensor 22 can be configured as a single axis sensor (SAS) comprising one coil as shown in Fig. 1. Similarly, body patch sensors 32 may also comprise similar coils.

The MTS typically assigns a first coordinate system, also referred to herein as a catheter frame of reference or a human coordinate system, for tracking the distal tip of the catheter relative to magnetic field generators 26. The MTS assigns a second. The MTS assigns another frame of reference (denoted herein as the patient frame of reference or sensor coordinate system) for monitoring the position of body patch sensors 32 relative to field generators 26. Body patch sensors 32 may also be referred to as back patch sensors as used in the CARTO system described above. In order to display the catheter position relative to the patient, the MTS typically registers the two frames of reference with one another.

If patient 17 moves on gurney 19 (and thus relative to field generators 26) during the operation of MTS 20, as detected by a change in position of body patch sensors 32, the catheter frame of reference shifts and should be subsequently updated by the measured change in the patient's frame of reference. Otherwise, the measured position of catheter distal tip 24 may no longer be accurate.

In some embodiments, a patient movement compensation system 30, connected to MTS 20, is configured to monitor patient movement, and to resynchronize the patient and catheter frames of reference. Since the MTS measures both the patient and catheter frames of reference, the two frames of reference are functionally related to one another, and in fact can be the same frame. The term "MTS frame of reference" can be used interchangeably herein to refer to either the patient or catheter frames of reference, or both. The MTS frame of reference is also referred to herein as the first coordinate system.

MTS 20 can accommodate small changes in the patient frame of reference within a "movement envelope" of the system due to the patient's movement. However when the patient movement exceeds a predefined threshold, by exceeding the movement envelope of the MTS, the MTS can no longer compensate for it.

In some embodiments of the present invention, when patient movement compensation system 30 detects that the patient movement exceeded the predefined threshold outside of the MTS movement envelope window, measurements from a fluoroscope imaging system (FIS) 40 are then used in conjunction with the MTS to resynchronize the MTS frame of reference described above without the need for an MTS recalibration cycle.

Initially, when MTS 20 is first calibrated as body patch sensors 32, which are opaque in the FIS, are placed on the surface of the patient body, a first fluoroscopic image is also acquired by a fluoroscopic detector 42 which is mounted above the patient. The first fluoroscopic image encompasses body patch sensors 32, the catheter in heart 28 and a hard tissue of the patient. For a cardiac procedure, the hard tissue typically comprises the rib cage, sternum bone, or spine of the patient.

The fluoroscopic image may comprise a full three-dimensional image model constructed from one or more fluoroscopic sub-images, e.g., single fluoroscopic image "snapshots." Typically in the CARTO system as an example embodiment, the first fluoroscopic image comprises a pair of images from two different angles, e.g., left anterior oblique (LAO) and right anterior oblique (RAO) images, which together enable three-dimensional reconstruction of the imaged volume, including the hard tissue.

The images can be viewed on output display monitor 50 by operator 70 along with the images or other cardiac signals from the MTS, such as an electrocardiogram signal and other information about the cardiac procedure for the operator.

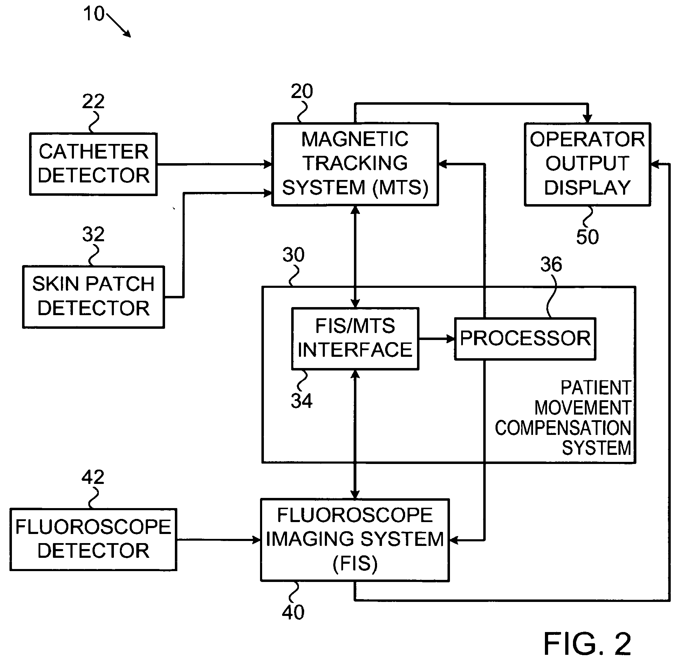

Fig. 2 is a block diagram that schematically illustrates tracking system 10, in accordance with an embodiment of the present invention. Intra-body probe tracking system 10 comprises patient movement compensation system 30. Compensation system 30 further comprises an FIS/MTS interface 34 and a processor 36. Interface 34 is configured to receive, or acquire, measurement data of the catheter and body surface probe positions from MTS 20 and the fluoroscopic images from FIS 40. Information from both MTS 20 and FIS 40 can be observed on operator output display 50. Processor 36 may also relay information back to MTS 20 and FIS 40. In the block diagram of Fig. 2, catheter detector 22 and skin patch detectors 32 are the signal inputs to MTS 20, whereas fluoroscope detector 42 provides the received fluoroscopic image data to FIS 40.

The system and PMCS configurations shown in Figs. 1 and 2 are example configurations, which are shown purely for the sake of conceptual clarity. In alternative embodiments, any other suitable configuration may be used. Some elements of system 30 may be implemented in hardware, e.g., in one or more Application-Specific Integrated Circuits (ASICs) or Field-Programmable Gate Arrays (FPGAs). Additionally or alternatively, some elements of system 30 can be implemented using software, or using a combination of hardware and software elements. In some embodiments, processor 36 comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

Initially, processor 36 defines a second coordinate system, also referred to as an image frame of reference, using the positions of body patch sensors 32 from the first fluoroscopic image. Typically, processor 36 identifies the positions of body patch sensors in the first fluoroscopic image, e.g., the LAO and RAO images described previously. Using this identification, processor 36 is able to register the first image (the second coordinate system) with the MTS frame of reference (first coordinate system).

If processor 36 detects that the patient movement exceeded the predefined threshold outside of the MTS movement envelope window, a subsequent fluoroscope image is acquired by FIS 40 and provided to processor 36 via interface 34. The movement of the patient is then measured by processor 36 from the FIS data by comparing the position of the hard tissue in the first and subsequent fluoroscopic images as described below. Processor 36 then corrects the position measurements of the MTS (in the first coordinate system) so as to compensate for the patient movement (measured in the second coordinate system).

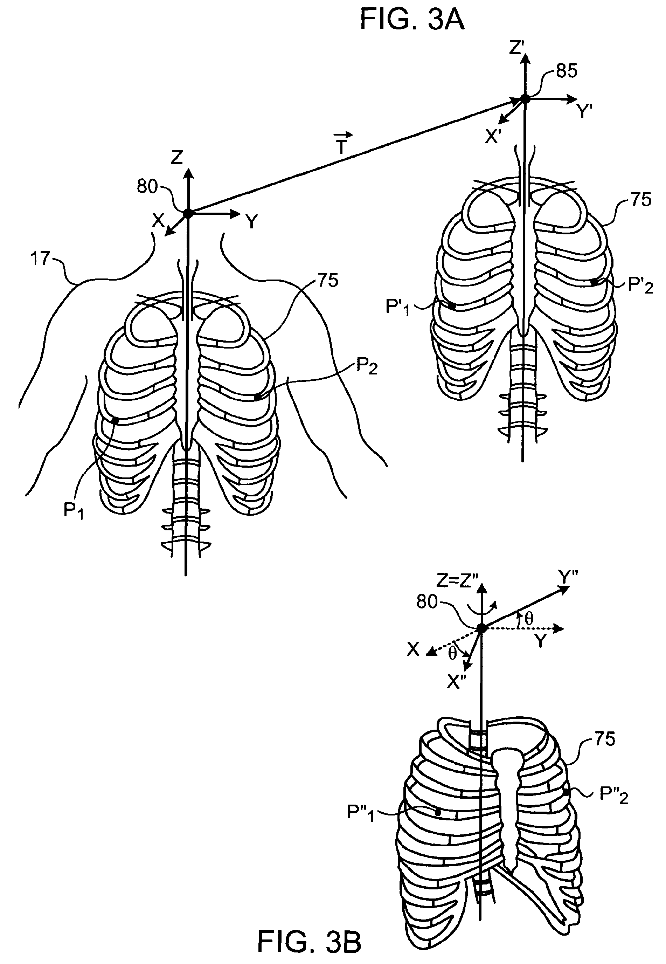

Figs. 3A and 3B are diagrams illustrating a translation and a rotation of hard tissue utilized in an intra-body probe tracking system, in accordance with an embodiment of the present invention. In the present context, the term "hard tissue" refers to a tissue type that is mechanically rigid and not flexible, such as bone. A change in the position of such hard tissue between different fluoroscopic images is thus indicative of movements of the patient. Hard tissue typically moves en-bloc, and its movement is therefore simpler to identify and measure.

Processor 36 assigns a first set of anchor points, such as P₁ and P₂, on the hard tissue to the first image. In the present example the hard tissue comprises a rib cage 75 within the chest of patient 17 for cardiac procedures as shown in Fig. 3A. The position of anchor points P₁ and P₂ are referenced to an origin 80 of a Cartesian coordinate system (X,Y,Z) by patient movement compensation system 30.

If the patient moves on the gurney laterally, the origin of (X,Y,Z) is shifted to a new origin 85 of coordinate system (X',Y',Z') as defined by a translation vector *̅T̅*̅ as shown in Fig. 3A. Translation vector *̅T̅*̅ can also be used to relate the anchor points P₁ and P₂ to the translated anchor points P'₁ and P'₂ of the hard tissue in the coordinate system (X',Y',Z'). Stated differently, (X,Y,Z) and (X',Y',Z') are both representations of the second (image) coordinate system with coordinate transformation defined by translation vector *̅T̅*̅.

Similarly, if the patient movement is a rotation about the axis through the spine while lying on the gurney, origin 80 of the coordinate system (X,Y,Z) remains the same in the rotated coordinate system (X",Y",Z") where Z"=Z. The rotation of the body can be expressed as a rotation of angle θ of the X,Y axes to the X",Y" axes in the rotated coordinate system as shown in Fig 3B. Similarly, rotation θ can also be used to relate the anchor points P₁ and P₂ to the rotated anchor points P"₁ and P"₂ on the hard tissue as measured by FIS 40. Stated differently, (X,Y,Z) and (X",Y",Z") are both representations of the second image coordinate system with coordinate transformation defined by rotation θ. Similar rotations can be measured relative to the other axes of the second coordinate system, to produce a rotation vector *̅θ̅*̅.

The patient movement on the gurney is typically a superposition of both a translation and a rotation operating on (X,Y,Z) with a coordinate transformation defined by the translation vector *̅T̅*̅ and rotation vector *̅θ̅*̅ of the hard tissue. Processor 36 then relays this data from the second coordinate system back to MTS 20, and MTS 20 applies the coordinate transformation defined by the translation vector *̅T̅*̅ and rotation *̅θ̅*̅ to the first (MTS) coordinate system to resynchronize the MTS frame of reference with the patient movement computed from the FIS frame. This correction restores the accuracy of the measured position of the distal tip of catheter within the patient body during the therapeutic procedure by the MTS without the need to recalibrate the MTS.

The fluoroscopic image of the hard tissue typically comprises two or more fluoroscopic sub-images received successively in order to create a three-dimensional (3D) model of the anatomical structure for analysis by operator 70. The anchor points are placed on the hard tissue on various landmarks of the anatomical structure, such as a joint, bend, or junction in the bones of the rib cage, which can then be easily identified by system 30 from the images after translation and rotation. Typically, digital fluoroscopic imaging processing techniques are used to resolve the landmarks.

In some embodiments, a first set of anchor points are defined on a first fluoroscopic three-dimensional model of the hard tissue, which is constructed by receiving, or acquiring, two fluoroscopic sub-images at different angles. A second subsequent fluoroscopic three-dimensional model comprises receiving, or acquiring, two sub-images at different angles on which the second set of anchor points are defined. The rotation *̅θ̅*̅ and the translation vector *̅T̅*̅ are computed from the change of the position anchor points between the first and second sets.

In other embodiments, system 10 can be configured to create an initial three-dimensional (3D) fluoroscopic model of the hard tissue and to generate a reconstructed 3D model by applying single fluoroscopic images. A first 3D reconstruction of the hard tissue can be made from a first fluoroscopic single image on which a first set of anchor points are defined. A second 3D reconstruction of the hard tissue can be made from a second subsequent fluoroscopic single image on which a second set of anchor points are defined. The rotation *̅θ̅*̅ and the translation vector *̅T̅*̅ are computed from the change of the position anchor points between the first and second sets, which are subsequently applied to the MTS frame of reference to correct patient movement on the gurney in the position measurement of the catheter distal tip.

The embodiments shown in Figs. 3A and 3B are purely for conceptual clarity and not by way of limitation of the embodiments of the present invention. For example, PMCS 30 may define one or more anchor points on the hard tissue in addition to P₁ and P₂. Any suitable coordinate transformation operating on the FIS image coordinate system (X,Y,Z) in addition to the translation vector and rotation as shown may be applied by PMCS 30 to correct the MTS frame of reference for the patient movement. The FIS using the image frame of reference (X,Y,Z) may include any suitable hard tissue landmarks in addition to points on the rib cage to measure the patient movement.

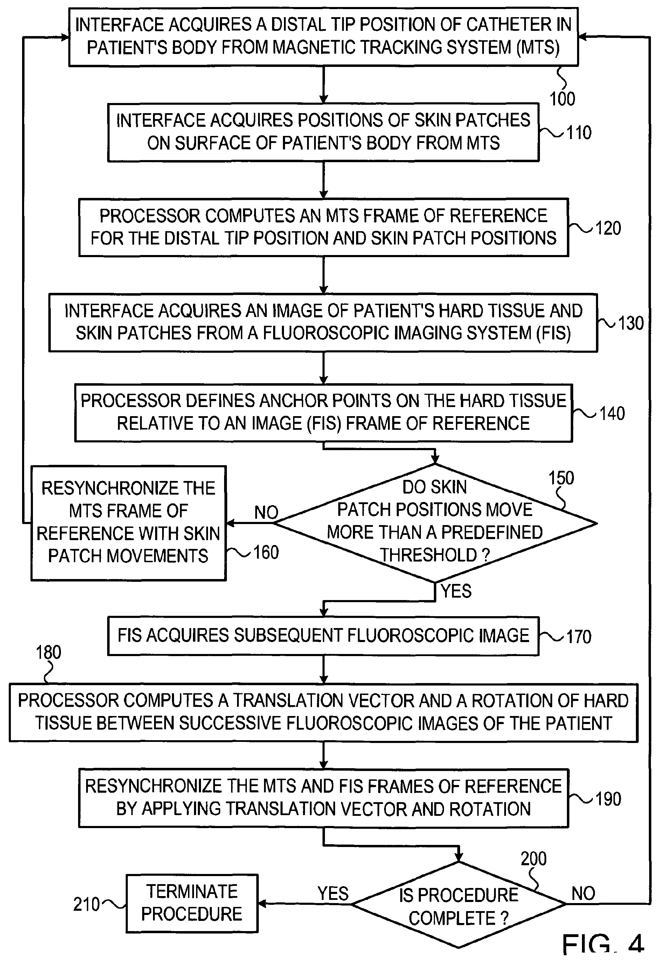

Fig. 4 is a flow chart that schematically illustrates a method for compensating for patient movement by intra-body probe tracking system 10, in accordance with an embodiment of the present invention. In a first acquiring step 100, FIS/MTS interface 34 acquires distal tip 24 position of catheter 15 in patient's body 17 from magnetic tracking system (MTS). In a second acquiring step 110, FIS/MTS interface 34 acquires positions of skin patch probe sensors 32 on surface of patient's body 17 from MTS. In a first computing step 120, processor 36 computes an MTS frame of reference for the distal tip position and skin patch positions. The MTS frame of reference, which can be represented using a first coordinate system, may relate the two dependent patient and catheter frames of reference, or may be the same as one of the frames as described previously.

In a third acquiring step 130, FIS/MTS interface 34 acquires an image of hard tissue 78 of patient and skin patch positions from fluoroscopic imaging system 40 (FIS). In a defining step 140, processor 36 defines anchor points on the hard tissue relative to a second coordinate system (X,Y,Z), e.g., image frame of reference as shown in Figs. 3A and 3B.

In a first decision step 150, if the MTS detects that the skin patch probe positions did not move more than a predefined threshold, e.g., outside of the movement envelope window, due to the patient movement on the gurney during the procedure, patient movement compensation system 30 resynchronizes the MTS frame of reference with the skin patch movements in a first resynchronizing step 160. The system then continues to monitor patient movement in first acquiring step 100.

If the patient moved more than the predefined threshold in decision step 150, FIS 40 acquires a subsequent fluoroscopic image 170 in a fourth acquiring step 170. In a second computing step 180, processor 36 computes a translation vector and rotation of hard tissue between successive fluoroscopic images of the patient as shown in Figs. 3A and 3B. The movement of the anchor points between successive fluoroscopic images is used to compute the translation vector and rotation of hard tissue in the FIS frame of reference. In a second resynchronizing step 190, patient movement compensation system 30 resynchronizes the MTS and FIS frames of reference by applying the translation vector and rotation to the MTS frame of reference. In other words, applying the computed translation vector and rotation of hard tissue from the FIS frame of reference to the MTS frame of reference is used to correct the position measurement of the catheter distal tip by the MTS for the patient's movement on the gurney.

In a second decision step 200, if intra-body probe tracking system 10 assesses that the therapeutic procedure is complete, the procedure is terminated in a terminating step 210. If not, patient movement compensation system 30 continues to monitor patient movement in first acquiring step 100.

To further improve the accuracy of the distal tip position measurement in the PMCS, a more accurate position measurement of the heart can be obtained by defining multiple anchor points in the heart itself, in addition to the anchor points defined on the skeletal hard tissue as shown in Figs. 3A and 3B. Tracking the movement of the heart anchor points can be used by processor 36 (in addition to the body patch positions) to improve the accuracy during the resynchronization of the MTS frame of reference due to patient movement, even if the patient movements may be small.

In some embodiments, heart anchor points can be defined while locating the position of the catheter distal tip at known landmarks in the heart, such as the right ventricular apex (RVA) and left atrial pulmonary veins. In other embodiments, additional heart anchor points may be obtained from anatomical points in the heart, such as the left ventricular apex (LVA) and heart valves, identified in the fluoroscopic images.

Alternatively, a more accurate catheter position measurement can be obtained from other imaging techniques used to identify additional heart anchor points. In some embodiments, heart anchor points can be defined by processor 36 as identified from landmark anatomical structures in the heart from images generated by rotational angiography. In other embodiments, heart anchor points can be defined by processor 36 as identified from landmark anatomical structures in the heart from ultrasound images generated by ultrasound catheter transesophageal echocardiography (TEE). Although the embodiments described herein mainly address catheter position tracking in cardiac procedures, the methods and systems described herein can also be used in other applications, such as catheter tracking in the bladder of the urinary tract, or in the stomach.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

Aspects of the invention not yet claimed:
Aspect 1. A method, comprising:
   receiving a position of an intra-body probe inserted into an organ of a living body in a first coordinate system;
   receiving fluoroscopic images of the body;
   measuring a movement of the body in the fluoroscopic images in a second coordinate system; and
   correcting the received position of the intra-body probe in the first coordinate system using the movement identified in the second coordinate system.
Aspect 2. The method according to aspect 1, wherein measuring the movement comprises identifying a hard tissue of the body in the fluoroscopic images, and assessing the movement of the hard tissue between a first fluoroscopic image and a subsequent fluoroscopic image.
Aspect 3. The method according to aspect 2, wherein assessing the movement comprises defining multiple anchor points on the hard tissue, and assessing the movement of the anchor points between the first fluoroscopic image and the subsequent fluoroscopic image.
Aspect 4. The method according to aspect 3, wherein correcting the received position of the intra-body probe comprises applying the movement of the anchor points, which was assessed in the second coordinate system, to the received position of the intra-body probe in the first coordinate system.
Aspect 5. The method according to aspect 1, wherein receiving the position comprises defining the first coordinate system relative to one or more body patch sensors using a magnetic tracking system, and wherein receiving the fluoroscopic images comprises identifying the body patch sensors in the fluoroscopic images and defining the second coordinate system relative to the identified body patch sensors.
Aspect 6. The method according to aspect 1, wherein correcting the received position using the measured movement of the body is performed upon determining that the movement of one or more body patch sensors, which are disposed on the body and detected by a magnetic tracking system, exceeds a predefined threshold.
Aspect 7. The method according to aspect 1, wherein receiving the fluoroscopic images comprises, for a given fluoroscopic image, receiving two or more fluoroscopic sub-images acquired at different angles in the second coordinate system.
Aspect 8. The method according to aspect 1, wherein measuring the movement of the body comprises constructing first and second three-dimensional models of hard tissue of the body from respective first and second fluoroscopic images, and measuring the movement of the hard tissue between the first and second three-dimensional models of the hard tissue.
Aspect 9. The method according to aspect 1, and further comprising tracking a movement of heart anchor points, so as to improve an accuracy in correcting the received position of the intra-body probe.

## Claims

1. An apparatus (30), comprising:
an interface (34), which is configured to receive a position of an intra-body probe (15) inserted into an organ of a living body in a first coordinate system, and to receive fluoroscopic images of the living body; and
a processor (36)
**characterised in that:**
the processor (36) is configured to measure a movement of the body in the fluoroscopic images in a second coordinate system, and to correct the received position of the intra-body probe (15) in the first coordinate system using the movement identified in the second coordinate system.

2. The apparatus according to claim 1, wherein the processor (36) is configured to measure the movement by identifying a hard tissue of the body in the fluoroscopic images, and assessing the movement of the hard tissue between a first fluoroscopic image and a subsequent fluoroscopic image.

3. The apparatus according to claim 2, wherein the processor (36) is configured to assess the movement by defining multiple anchor points on the hard tissue, and assessing the movement of the anchor points between the first fluoroscopic image and the subsequent fluoroscopic image.

4. The apparatus according to claim 3, wherein the processor (36) is configured to correct the received position of the intra-body probe (15) by applying the movement of the anchor points, which was assessed in the second coordinate system, to the received position of the intra-body probe (15) in the first coordinate system.

5. The apparatus according to claim 1, wherein the processor (36) is configured to define the first coordinate system relative to one or more body patch sensors (32) of a magnetic tracking system (20), to identify the body patch sensors (32) in the fluoroscopic images and to define the second coordinate system relative to the identified body patch sensors (32).

6. The apparatus according to claim 1, wherein the processor (36) is configured to correct the received position using the measured movement of the body upon determining that the movement of one or more body patch sensors (32), which are disposed on the body and detected by a magnetic tracking system (20), exceeds a predefined threshold.

7. The apparatus according to claim 1, wherein the interface (34) is configured to receive, for a given fluoroscopic image, two or more fluoroscopic sub-images acquired at different angles in the second coordinate system.

8. The apparatus according to claim 1, wherein the processor (36) is configured to measure the movement of the body by constructing first and second three-dimensional models of hard tissue of the body from respective first and second fluoroscopic images, and measuring the movement of the hard tissue between the first and second three-dimensional models of the hard tissue.

9. The apparatus according to claim 1, wherein the processor (36) is configured to track a movement of heart anchor points, so as to improve an accuracy in correcting the received position of the intra-body probe.

10. An apparatus, comprising:
a magnetic intra-body probe tracking system (20);
a fluoroscopic imaging system (40); and
a patient movement compensation system (30) comprising the apparatus of claim 1.

## Patentansprüche

1. Vorrichtung (30), umfassend:
eine Schnittstelle (34), die dafür gestaltet ist, eine Position einer Intrakorporalsonde (15), die in ein Organ eines lebenden Körpers eingeführt ist, in einem ersten Koordinatensystem zu empfangen und fluoroskopische Bilder des lebenden Körpers zu empfangen; und
einen Prozessor (36),
**dadurch gekennzeichnet, dass:**
der Prozessor (36) dafür gestaltet ist, eine Bewegung des Körpers in den fluoroskopischen Bildern in einem zweiten Koordinatensystem zu messen und die empfangene Position der Intrakorporalsonde (15) in dem ersten Koordinatensystem unter Verwendung der in dem zweiten Koordinatensystem identifizierten Bewegung zu korrigieren.

2. Vorrichtung gemäß Anspruch 1, wobei der Prozessor (36) dafür gestaltet ist, die Bewegung durch Identifizieren eines harten Gewebes des Körpers in den fluoroskopischen Bildern und Bestimmen der Bewegung des harten Gewebes zwischen einem ersten fluoroskopischen Bild und einem nachfolgenden fluoroskopischen Bild zu messen.

3. Vorrichtung gemäß Anspruch 2, wobei der Prozessor (36) dafür gestaltet ist, die Bewegung durch Definieren mehrerer Ankerpunkte an dem harten Gewebe und Bestimmen der Bewegung der Ankerpunkte zwischen dem ersten fluoroskopischen Bild und dem nachfolgenden fluoroskopischen Bild zu bestimmen.

4. Vorrichtung gemäß Anspruch 3, wobei der Prozessor (36) dafür gestaltet ist, die empfangene Position der Intrakorporalsonde (15) durch Anwenden der Bewegung der Ankerpunkte, die in dem zweiten Koordinatensystem bestimmt wurde, auf die empfangene Position der Intrakorporalsonde (15) in dem ersten Koordinatensystem zu korrigieren.

5. Vorrichtung gemäß Anspruch 1, wobei der Prozessor (36) dafür gestaltet ist, das erste Koordinatensystem relativ zu einem oder mehreren Körperpflastersensoren (32) eines magnetischen Ortungssystems (20) zu definieren, die Körperpflastersensoren (32) in den fluoroskopischen Bildern zu identifizieren und das zweite Koordinatensystem relativ zu den identifizierten Körperpflastersensoren (32) zu definieren.

6. Vorrichtung gemäß Anspruch 1, wobei der Prozessor (36) dafür gestaltet ist, die empfangene Position unter Verwendung der gemessenen Bewegung des Körpers bei Beobachtung, dass die Bewegung eines oder mehrerer Körperpflastersensoren (32), die an dem Körper angeordnet sind und von einem magnetischen Ortungssystem (20) nachgewiesen werden, einen vorbestimmten Schwellenwert überschreitet, zu korrigieren.

7. Vorrichtung gemäß Anspruch 1, wobei die Schnittstelle (34) dafür gestaltet ist, für ein gegebenes fluoroskopisches Bild zwei oder mehr fluoroskopische Teilbilder zu empfangen, die unter verschiedenen Winkeln in dem zweiten Koordinatensystem aufgenommen werden.

8. Vorrichtung gemäß Anspruch 1, wobei der Prozessor (36) dafür gestaltet ist, die Bewegung des Körpers durch Erstellen eines ersten und eines zweiten dreidimensionalen Modells von hartem Gewebe des Körpers aus entsprechenden ersten und zweiten fluoroskopischen Bildern und Messen der Bewegung des harten Gewebes zwischen dem ersten und dem zweiten dreidimensionalen Modell des harten Gewebes zu messen.

9. Vorrichtung gemäß Anspruch 1, wobei der Prozessor (36) dafür gestaltet ist, eine Bewegung von Herz-Ankerpunkten zu orten, um die Genauigkeit der Korrektur der empfangenen Position der Intrakorporalsonde zu verbessern.

10. Vorrichtung, umfassend:
ein magnetisches Intrakorporalsonden-Ortungssystem (20);
ein fluoroskopisches Bildgebungssystem (40); und
ein Patientenbewegungs-Kompensationssystem (30), das die Vorrichtung gemäß Anspruch 1 umfasst.

## Revendications

1. Appareil (30), comprenant :
une interface (34), qui est configurée pour recevoir une position d'une sonde intracorporelle (15) insérée dans un organe d'un corps vivant dans un premier système de coordonnées, et pour recevoir des images fluoroscopiques du corps vivant ; et
un processeur (36),
**caractérisé en ce que** :
le processeur (36) est configuré pour mesurer un mouvement du corps dans les images fluoroscopiques dans un deuxième système de coordonnées, et pour corriger la position reçue de la sonde intracorporelle (15) dans le premier système de coordonnées en utilisant le mouvement identifié dans le deuxième système de coordonnées.

2. Appareil selon la revendication 1, dans lequel le processeur (36) est configuré pour mesurer le mouvement en identifiant un tissu dur du corps dans les images fluoroscopiques, et en évaluant le mouvement du tissu dur entre une première image fluoroscopique et une image fluoroscopique suivante.

3. Appareil selon la revendication 2, dans lequel le processeur (36) est configuré pour évaluer le mouvement en définissant de multiples points d'ancrage sur le tissu dur, et en évaluant le mouvement des points d'ancrage entre la première image fluoroscopique et l'image fluoroscopique suivante.

4. Appareil selon la revendication 3, dans lequel le processeur (36) est configuré pour corriger la position reçue de la sonde intracorporelle (15) en appliquant le mouvement des points d'ancrage, qui a été évalué dans le deuxième système de coordonnées, à la position reçue de la sonde intracorporelle (15) dans le premier système de coordonnées.

5. Appareil selon la revendication 1, dans lequel le processeur (36) est configuré pour définir le premier système de coordonnées par rapport à un ou plusieurs capteurs corporels de type timbre (32) d'un système de suivi magnétique (20), pour identifier les capteurs corporels de type timbre (32) dans les images fluoroscopiques et pour définir le deuxième système de coordonnées par rapport aux capteurs corporels de type timbre (32) identifiés.

6. Appareil selon la revendication 1, dans lequel le processeur (36) est configuré pour corriger la position reçue en utilisant le mouvement mesuré du corps lorsqu'il est déterminé que le mouvement d'un ou plusieurs capteurs corporels de type timbre (32), qui sont disposés sur le corps et détectés par un système de suivi magnétique (20), dépasse un seuil prédéfini.

7. Appareil selon la revendication 1, dans lequel l'interface (34) est configurée pour recevoir, pour une image fluoroscopique donnée, au moins deux sous-images fluoroscopiques acquises à des angles différents dans le deuxième système de coordonnées.

8. Appareil selon la revendication 1, dans lequel le processeur (36) est configuré pour mesurer le mouvement du corps en construisant des premier et deuxième modèles tridimensionnels de tissu dur du corps à partir de première et deuxième images fluoroscopiques respectives, et en mesurant le mouvement du tissu dur entre le premier et le deuxième modèle tridimensionnel du tissu dur.

9. Appareil selon la revendication 1, dans lequel le processeur (36) est configuré pour suivre un mouvement de points d'ancrage cardiaques, de manière à améliorer une précision dans la correction de la position reçue de la sonde intracorporelle.

10. Appareil, comprenant :
un système de suivi de sonde intracorporelle magnétique (20) ;
un système d'imagerie fluoroscopique (40) ; et
un système de compensation de mouvement de patient (30) comprenant l'appareil de la revendication 1.
